# DEMANDE DE BREVET EUROPEEN

(11) **EP 1 889 604 A2**
(43) Date de publication de la demande: **20.02.2008**
(21) Numéro de dépôt: 07301200.7
(22) Date de dépôt: 02.07.2007
(51) Int. Cl.: A61K 8/60, A61Q 19/00, A61K 31/70

(54) **Utilisation d'un dérivé C-glycoside pour améliorer la fonction barrière de la peau**

(30) Priorité: 03.07.2006 FR 0606025
(71) Demandeur: L'ORÉAL, 75008 Paris (FR)
(72) Inventeur: Burnier, Véronique, 91210, Draveil (FR); Besne, Isabelle, 92800, Puteaux (FR)
(74) Mandataire: Le Coupanec, Pascale A.M.P.

(57) **Abrégé**

La présente invention concerne l'utilisation cosmétique d'au moins un dérivé C-glycoside dans une composition, pour améliorer et/ou renforcer la fonction barrière de la peau.

## Description

La présente invention concerne l'utilisation notamment cosmétique d'au moins un dérivé C-glycoside dans une composition ou pour la préparation d'une composition, ledit dérivé ou ladite composition étant destiné(e) à améliorer et/ou renforcer la fonction barrière de la peau. Elle porte aussi sur un procédé de traitement cosmétique de la peau destiné à améliorer la fonction barrière comprenant au moins une étape consistant à appliquer sur la peau au moins une composition comprenant au moins un dérivé C-glycoside.

La peau humaine est constituée de deux compartiments, à savoir un compartiment profond, le derme et un compartiment superficiel, l'épiderme.

Elle constitue une barrière contre les agressions extérieures, notamment chimiques, mécaniques ou infectieuses, et à ce titre un certain nombre de réactions de défense contre les facteurs environnementaux (climat, rayons ultraviolets, tabac, ...) et/ou les xénobiotiques, comme par exemple les micro-organismes, se produisent à son niveau. Cette propriété, appelée fonction barrière, est principalement assurée par la couche la plus superficielle de l'épiderme, à savoir la couche cornée, appelée le *stratum corneum.*

Les cellules constituant l'épiderme (majoritairement les kératinocytes, mais aussi les mélanocytes et les cellules de Langerhans) sont délimitées par une structure lipidique intercellulaire. Chacun de ces types cellulaires contribue par ses fonctions propres au rôle essentiel joué dans l'organisme par la peau. En particulier, les kératinocytes subissent un processus de maturation continu et orienté qui, des kératinocytes se trouvant dans la couche basale de l'épiderme, aboutit à la formation de cornéocytes, qui sont des cellules mortes totalement kératinisées constituées de kératinocytes au stade terminal de leur différenciation.

Au cours de la différenciation, les phospholipides dont le rôle consiste à élaborer la structure fluide des membranes cellulaires des couches vivantes de l'épiderme, sont peu à peu remplacés par un mélange composé en majeure partie d'acides gras, de cholestérol et de sphingolipides (céramides). Ces lipides, qui sont organisés en phases cristal liquide lamellaires spécifiques, forment le ciment intracellulaire du *stratum corneum* et sont essentiels pour les échanges en eau et la fonction barrière de l'épiderme. Ainsi, la structure lamellaire des lipides du domaine lipidique de l'épiderme et les cornéocytes participent à la fonction barrière épidermique.

Il est manifeste que la qualité de la barrière cutanée et des muqueuses est dépendante de mécanismes biologiques endogènes complexes faisant intervenir de nombreux facteurs de croissance, des molécules d'adhésion, des hormones et des enzymes du métabolisme lipidique.

Ainsi une altération de la barrière cutanée peut se produire en présence d'agressions externes de type agents irritants (détergents, acides, bases, oxydants, réducteurs, solvants concentrés, gaz ou fumées toxiques), sollicitations mécaniques (frottements, chocs, abrasion, arrachement de la surface, projection de poussières, de particules, rasage ou épilation), déséquilibres thermiques ou climatiques (froid, sécheresse, radiations), xénobiotiques (micro-organismes indésirable, allergènes) ou d'agressions internes de type stress psychologique.

Cette altération de la barrière cutanée peut notamment se traduire par un inconfort cutané, des phénomènes sensoriels et notamment des phénomènes désagréables. Les terminaisons nerveuses sensorielles de la surface de la peau ou du cuir chevelu, notamment les fibres C des neurones, les récepteurs ou capteurs chimiques, thermiques, mécaniques ou les barorécepteurs sont en effet partiellement "dénudées". L'homme peut alors éprouver une sensation d'inconfort cutané qui peut se manifester notamment par des picotements, des tiraillements, des échauffements, des démangeaisons.

Ces sensations d'inconfort cutané sont plus fréquentes dans les zones les plus exposées de l'organisme, à savoir les mains, les pieds, le visage, et le cuir chevelu.

Elles peuvent survenir notamment sur des zones soumises à certains gestes d'hygiène quotidienne ou fréquemment renouvelés tels que le rasage, l'épilation, la détersion par des produits de toilette ou des produits ménagers, l'application d'adhésifs (pansements, patchs, fixation de prothèses) ou dans le cas de gestes sportifs, professionnels
ou simplement liés au mode de vie et à l'utilisation de vêtements, d'outils ou d'équipements générant des frottements localisés. Elles peuvent également être amplifiées par le stress psychologique.

Ces sensations d'inconfort cutané concernent toutes les personnes, et en particulier :
- les personnes à peau dite 'fragile' ou 'délicate' et vulnérable se déséquilibrant rapidement lors de variations de la température ou de l'humidité relative de grande amplitude (cas des peaux de bébé par exemple) ;
- les personnes à peau dite 'fragilisée', regroupant notamment
   - les personnes dont le film hydro-lipidique protecteur composé de sueur, de sébum et de facteurs d'hydratation naturelle se raréfie, comme c'est le cas pour les personnes âgées de plus de 60 ans et notamment dans le cadre du grand âge (au moins 75 ans) ;
   - les personnes dont la composition du film hydro-lipidique est modifiée, comme c'est le cas des personnes diabétiques, ou dialysées, ou atteintes de certaines maladies ;
- les personnes qui possèdent une seuil de réactivité abaissé du à une hyperactivité neurogène ; ces peaux vont donc présenter ces sensations et ces signes cliniques beaucoup plus rapidement et fréquemment que les autres types de peaux : ce sont les personnes à peaux sensibles.

On pourra également parler de personnes à peau 'agressée' pour les peaux rasées par exemple.

On sait par ailleurs que les peaux atopiques peuvent être liées à une diminution de la synthèse des céramides 1 et/ou 3, comme cela est notamment rapporté dans les documents suivants:
- Imokawa et al. dans J Invest Dermatol, 96 (4): 523-6, 1991, qui décrivent que les céramides interviennent dans la fonction barrière et que leur synthèse, en particulier celle du céramide 1, est diminuée dans le cas des peaux atopiques et xérotiques.
- Di Nardo et al. dans Acta Derm Venereol, 78 (1) : 27-30, (1998) qui décrivent la dermite atopique comme une peau facilement irritable et sèche, dans laquelle la fonction barrière est altérée.

On recherche donc à améliorer et/ou renforcer la fonction barrière cutanée pour :
- prévenir et/ou diminuer les sensations d'inconfort cutané, de picotements, tiraillements, échauffements et démangeaisons, en particulier chez les personnes à peau fragile ou délicate (par exemple les bébés) ; ou les personnes à peau fragilisée (telles que les personnes âgées d'au moins 60 ans et en particulier les personnes d'au moins 75 ans), ou les personnes dont la composition du film hydro-lipidique est modifiée, comme c'est le cas des personnes diabétiques, ou dialysées, ou atteintes de certaines maladies, ou les personnes à peaux sensibles,
- et/ou améliorer la fonction barrière cutanée de peaux atopiques et/ou prolonger les phases de rémission entre des crises aiguës de ce type d'affection.

Dans ce cadre, les inventeurs ont découvert que certains dérivés C-glycosides étaient capables d'améliorer et/ou de renforcer la fonction barrière de la peau.

Les sucres et dérivés du sucre sont des produits déjà mis à profit à des fins diverses pour la formulation de compositions cosmétiques destinées tant au soin de la peau qu'au soin et/ou au lavage des fibres kératiniques.

Ainsi, dans le document WO 99/24009, le D-xylose et ses dérivés sont proposés à des fins de préparation de produits cosmétiques ou pharmaceutiques visant à améliorer la fonctionnalité des cellules de l'épiderme.

Parmi les sucres utilisables dans le domaine, les dérivés C-glycosides s'avèrent tout particulièrement intéressants. Certains dérivés C-glycosides ont notamment démontré des propriétés biologiques intéressantes, en particulier pour lutter contre le vieillissement de l'épiderme et/ou contre le dessèchement de la peau. De tels composés sont notamment décrits dans le document WO 02/051828.

Ces composés agissent par l'intermédiaire de la stimulation de la synthèse des glycosaminoglycannes contenant un résidu D-glucosamine et/ou N-acétyl-D-glucosamine et sont représentés par la formule : 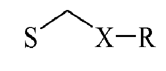 dans laquelle S représente un monosaccharide ou un polysaccharide, R représente différents radicaux linéaires ou cycliques et le groupement X peut représenter un groupement choisi parmi : -CO-, -CH(NR₁R₂)-, -CHR'-, -C(=CHR₃)- avec R₁, R₂, R' et R₃ pouvant représenter différent radicaux, dont le radical hydroxyle pour R₁, R₂ et R₃.

Toutefois, ces dérivés n'avaient jusqu'à présent jamais été utilisés à des fins d'amélioration et/ou de renforcement de la fonction barrière cutanée.

De par cette amélioration et/ou ce renforcement de la fonction barrière cutanée par l'application topique d'une composition selon l'invention, toutes les peaux et en particulier les peaux fragiles ou fragilisées (par exemple les peaux de bébé, de personnes d'au moins 60 ans de préférence d'au moins 75 ans de personnes diabétiques ou dialysées), et les peaux sensibles sont mieux protégées des agressions extérieures, chimiques, mécaniques, ou infectieuses.

Les inventeurs ont en particulier démontré sur un modèle de peau reconstruite une amélioration de la fonction barrière de la peau par diminution du passage d'une caféine radiomarquée après traitement de ladite peau pas lesdits composés C-glycosides.

L'invention concerne ainsi selon un premier de ses aspects, l'utilisation cosmétique d'au moins un dérivé C-glycoside dans une composition, pour améliorer et/ou renforcer la fonction barrière de la peau.

Elle concerne également, selon un autre de ses aspects, l'utilisation cosmétique d'au moins un dérivé C-glycoside dans une composition, pour améliorer et/ou renforcer la protection de la peau vis-à-vis d'agressions extérieures.

En particulier, ledit dérivé C-glycoside et/ou ladite composition peuvent être destinés à prévenir et/ou diminuer l'inconfort cutané d'une peau, en particulier induit par un stress exogène d'origine chimique, environnementale, mécanique et/ou un stress endogène, en particulier d'une peau fragile, fragilisée et/ou d'une peau sensible, telles que définies précédemment.

L'inconfort cutané peut être notamment caractérisé par des tiraillements, des picotements, des échauffements et/ou des démangeaisons.

Selon un autre mode de réalisation, ledit dérivé C-glycoside et/ou ladite composition peuvent être destinés à améliorer et/ou renforcer la fonction barrière d'une peau choisie parmi une peau fragile, une peau fragilisée, une peau agressée et/ou une peau sensible.

Dans le cadre de l'invention, les dérivés C-glycoside peuvent être utilisés pour l'application sur la peau saine, soumise ou pouvant être soumise à l'influence d'agents tels que des agents climatiques et de ce fait susceptible de manifester un inconfort cutané. Dans d'autres cas particuliers, les dérivés C-glycosides de l'invention peuvent être appliqués sur la peau lorsqu'elle présente des signes cliniques de déficit de la barrière cutanée, par exemple les peaux atopiques.

Ainsi l'invention a pour objet l'utilisation d'au moins un dérivé C-glycoside, pour la préparation d'une composition notamment dermatologique destinée à améliorer et/ou renforcer la fonction barrière de la peau.

Un autre aspect de la présente invention est donc l'utilisation d'au moins un dérivé C-glycoside, pour la préparation d'une composition notamment dermatologique destinée à améliorer et/ou renforcer la fonction barrière d'une peau lésée, en particulier d'une peau atopique.

La composition selon l'invention peut notamment être destinée à prolonger les phases de rémission entre des crises aiguës d'affections dermatologiques, par exemple de type atopie.

### DERIVES C-GLYCOSIDES

Un dérivé C-glycoside convenant à l'invention peut être un composé de formule générale (I) suivante : 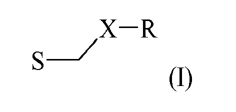 dans laquelle :
- R représente :
   - un radical alkyle linéaire, saturé en C₁ à C₂₀, en particulier en C₁ à C₁₀, ou insaturé en C₂ à C₂₀, en particulier en C₂ à C₁₀, ou un radical alkyle ramifié ou cyclique, saturé ou insaturé, en C₃ à C₂₀, en particulier en C₃ à C₁₀ ;
   - un radical hydrofluoro- ou perfluoro-alkyle, linéaire saturé en C₁ à C₂₀, en particulier en C₁ à C₁₀, ou insaturé en C₂ à C₂₀, en particulier en C₂ à C₁₀, ou ramifié ou cyclique, saturé ou insaturé, en C₃ à C₂₀, en particulier en C₃ à C₁₀ ;
la chaîne hydrocarbonée constituant lesdits radicaux pouvant, le cas échéant, être interrompue par 1, 2, 3 ou plus d'hétéroatomes choisis parmi :
- un oxygène,
- un soufre,
- un azote, et
- un silicium,
et pouvant être éventuellement substituée par au moins un radical choisi parmi :
- -OR₄,
- -SR₄,
- -NR₄R₅,
- -COOR₄,
- -CONHR₄,
- -CN,
- un atome d'halogène,
- un radical hydrofluoro- ou perfluoro-alkyle, en C₁ à C₆, et/ou
- un radical cycloalkyle en C₃ à C₈,
avec R₄ et R₅ pouvant représenter, indépendamment l'un de l'autre, un atome d'hydrogène, ou un radical alkyle, perfluoroalkyle ou hydrofluoroalkyle linéaire, saturé en C₁ à C₃₀, notamment en C₁ à C₁₂, ou insaturé en C₂ à C₃₀, notamment en C₂ à C₁₂, ou ramifié ou cyclique, saturé ou insaturé, en C₃ à C₃₀, notamment en C₃ à C₁₂ ; ou un radical aryle en C₆ à C₁₀,
- X représente un radical choisi parmi les groupements : 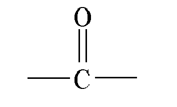 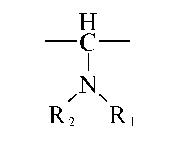 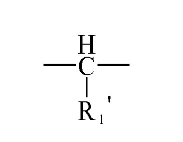 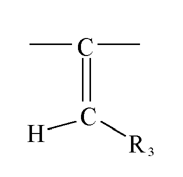 avec R₁, R₂ et R₃ représentant, indépendamment l'un de l'autre, un atome d'hydrogène, ou un radical R, avec R tel que défini précédemment, et R'₁ représente un atome d'hydrogène, un groupe -OH ou un radical R tel que défini précédemment, R₁ pouvant désigner également un radical aryle en C₆ à C₁₀;
- S représente un monosaccharide ou un polysaccharide comportant jusqu'à 20 unités sucre, en particulier jusqu'à 6 unités sucre, sous forme pyranose et/ou furanose et de série L et/ou D, ledit mono- ou poly-saccharide pouvant être substitué par un groupement hydroxyle obligatoirement libre, et éventuellement une ou plusieurs fonction(s) amine(s) éventuellement protégée(s), et
- la liaison S-CH₂-X représente une liaison de nature C-anomérique, qui peut être α ou β,
   ainsi que leurs sels cosmétiquement acceptables, leurs solvates tels que les hydrates et leurs isomères.

Dans le cadre de la présente invention, par « halogène » , on entend le chlore, le fluor, le brome ou l'iode.

Le terme « aryle »désigne un cycle aromatique tel que phényle, éventuellement substitué par un ou plusieurs radicaux alkyles en C₁-C₄.

Le terme « cycloalkyle en C₃ à C₈ »désigne un cycle aliphatique ayant de 3 à 8 atomes de carbone, incluant par exemple le cyclopropyle, le cyclopentyle et le cyclohéxyle.

Parmi les groupes alkyle convenant à la mise en oeuvre de l'invention, on peut notamment citer les groupes méthyle, éthyle, isopropyle, n-propyle, n-butyle, t-butyle, isobutyle, sec-butyle, pentyle, n-hexyle, cyclopropyle, cyclopentyle, cyclohexyle, et allyle.

Selon un mode de réalisation de l'invention, on peut utiliser un dérivé C-glycoside répondant à la formule (I) pour lequel S peut représenter un monosaccharide ou un polysaccharide contenant jusqu'à 6 unités sucre, sous forme pyranose et/ou furanose et de série L et/ou D, ledit mono- ou polysaccharide présentant au moins une fonction hydroxyle obligatoirement libre et/ou éventuellement une ou plusieurs fonctions amine obligatoirement protégée, X et R conservant par ailleurs l'ensemble des définitions précédemment données.

Avantageusement, un monosaccharide de l'invention peut être choisi parmi le D-glucose, le D-galactose, le D-mannose, le D-xylose, le D-lyxose, le L-fucose, le L-arabinose, le L-rhamnose, l'acide D-glucuronique, l'acide D-galacturonique, l'acide D-iduronique, la N-acétyl-D-glucosamine, la N-acétyl-D-galactosamine et désigne avantageusement le D-glucose, le D-xylose, la N-acétyl-D-glucosamine ou le L-fucose, et en particulier le D-xylose.

Plus particulièrement, un polysaccharide de l'invention contenant jusqu'à 6 unités sucre peut être choisi parmi le D-maltose, le D-lactose, le D-cellobiose, le D-maltotriose, un disaccharide associant un acide uronique choisi parmi l'acide D-iduronique ou l'acide D-glucuronique avec une hexosamine choisie parmi la D-galactosamine, la D-glucosamine, la N-acétyl-D-galactosamine, la N-acétyl-D-glucosamine, un oligosaccharide contenant au moins un xylose qui peut être avantageusement choisi parmi le xylobiose, le méthyl-β-xylobioside, le xylotriose, le xylotétraose, le xylopentaose et le xylohexaose et notamment le xylobiose qui est composé de deux molécules de xylose liées par une liaison 1-4.

Plus particulièrement, S peut représenter un monosaccharide choisi parmi le D-glucose, le D-xylose, le L-fucose, le D-galactose, le D-maltose et notamment le D-xylose.

Selon un autre mode de réalisation de l'invention, on peut utiliser des dérivés C-glycoside répondant à la formule (I) pour lesquels X représente un groupement choisi parmi -CO-, -CH(OH)-, -CH(NR₁R₂)-, -CH(R)-, en particulier -CO-, -CH(OH)-, -CH(NH₂)-, -CH(NHCH₂CH₂CH₂OH)-, -CH(NHPh)-, -CH(CH₃)-, et plus particulièrement un groupement -CO-, -CH(OH)-, -CH(NH₂)-, et préférentiellement un groupement -CH(OH)-, S et R conservant par ailleurs l'ensemble des définitions précédemment données.

Selon un autre mode de réalisation de l'invention, on peut utiliser un dérivé C-glycoside répondant à la formule (I) pour lesquels R représente un radical alkyle linéaire, saturé en C₁ à C₂₀, en particulier en C₁ à C₁₀, ou insaturé en C₂ à C₂₀, en particulier en C₂ à C₁₀, ou un radical alkyle ramifié ou cyclique, saturé ou insaturé, en C₃ à C₂₀ ; en particulier en C₃ à C₁₀, et éventuellement substitué comme décrit précédemment, S et X conservant par ailleurs l'ensemble des définitions précédemment données. De préférence, R désigne un radical linéaire en C₁-C₄, notamment C₁-C₃, éventuellement substitué par -OH, -COOH ou -COOR"₂, R"₂ étant un radical alkyle saturé en C₁-C₄, notamment éthyle.

Préférentiellement R désigne un radical alkyle linéaire non substitué en C₁-C₄, notamment C₁-C₂, en particulier éthyle.

Parmi les dérivés C-glycoside de formule (I), on utilise de préférence ceux pour lesquels :
- R représente un radical alkyle en linéaire, saturé en C₁ à C₂₀, en particulier en C₁ à C₁₀, ou insaturé en C₂ à C₂₀, en particulier en C₂ à C₁₀, ou un radical alkyle ramifié ou cyclique, saturé ou insaturé, en C₃ à C₂₀; en particulier en C₃ à C₁₀, et éventuellement substitué comme décrit précédemment ;
- S représente un monosaccharide comme décrit précédemment ;
- X représente -CO-, -CH(OH)-, -CH(NR₁R₂)-, -CH(R)- comme décrit précédemment.

De préférence, on utilise un dérivé C-glycoside de formule (I) pour lesquels :
- R désigne un radical linéaire en C₁-C₄, notamment C₁-C₃, éventuellement subsitué par -OH, -COOH ou -COOR"₂, R"₂ étant un radical alkyle saturé en C₁-C₄, notamment éthyle ;
- S représente un monosaccharide comme décrit précédemment ;
- X représente un groupement choisi parmi -CO-, -CH(OH)-, -CH(NH₂)-, -CH(NHCH₂CH₂CH₂OH)-, -CH(NHPh)-, -CH(CH₃)-, et plus particulièrement un groupement -CO-, -CH(OH)-, -CH(NH₂)-, et préférentiellement un groupement -CH(OH)-.

Préférentiellement, on utilise un dérivé C-glycoside de formule (I) pour lesquels :
- R désigne un radical alkyle linéaire non substitué en C₁-C₄, notamment C₁-C₂, en particulier éthyle ;
- S représente un monosaccharide comme décrit précédemment ; notamment le D-glucose, le D-xylose, la N-acétyl-D-glucosamine ou le L-fucose, et en particulier le D-xylose ;
- X représente un groupement choisi parmi -CO-, -CH(OH)-, -CH(NH₂)-, et préférentiellement un groupement -CH(OH)- .

Les sels acceptables pour l'usage non thérapeutique des composés décrits dans la présente invention comprennent des sels non toxiques conventionnels desdits composés tels que ceux formés à partir d'acides organiques ou inorganiques. A titre d'exemple, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Lorsque le composé de formule (I) comporte un groupe acide, la neutralisation du ou des groupes acides peut être effectuée par une base minérale, telle que LiOH, NaOH, KOH, Ca(OH)₂, NH₄OH, Mg(OH)₂ ou Zn(OH)₂; ou par une base organique telle qu'une alkylamine primaire, secondaire ou tertiaire, par exemple la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine, la diméthylamino-2-propanol, le 2-amino-2-(hydroxyméthyl)-1,3-propanediol. On peut encore citer la lysine ou la 3-(diméthylamino)propylamine.

Les solvates acceptables pour les composés décrits dans la présente invention comprennent des solvates conventionnels tels que ceux formés lors de la dernière étape de préparation desdits composés du fait de la présence de solvants. A titre d'exemple, on peut citer les solvates dus à la présence d'eau ou d'alcools linéaires ou ramifiés comme l'éthanol ou l'isopropanol.

Parmi les dérivés C-glycosides de formule (I), utilisés selon l'invention, on considère tout particulièrement :
- 1.: C-β-D-xylopyranoside-n-propane-2-one ;
- 2.: C-α-D-xylopyranoside-n-propane-2-one ;
- 3.: 1-[2-(3-hydroxy-propylamino)-propyl]-C-β-D-xylopyranose ;
- 4.: 1-[2-(3-hydroxy-propylamino)-propyl]-C-α-D-xylopyranose ;
- 5.: C-β-D-xylopyranoside-2-hydroxy-propane ;
- 6.: C-α-D-xylopyranoside-2-hydroxy-propane ;
- 7.: C-β-D-xylopyranoside-2-amino-propane ;
- 8.: C-α-D-xylopyranoside-2-amino-propane ;
- 9.: C-β-D-xylopyranoside-2-phénylamino-propane ;
- 10.: C-α-D-xylopyranoside-2-phénylamino-propane ;
- 11.: ester éthylique de l'acide 3-méthyl-4-(C-β-D-xylopyranoside)-butyrique ;
- 12.: ester éthylique de l'acide 3-méthyl-4-(C-α-D-xylopyranoside)-butyrique ;
- 13.: acide 6-(C-β-D-xylopyranoside)-5-céto-hexanoique ;
- 14.: acide 6-(C-α-D-xylopyranoside)-5-céto-hexanoique ;
- 15.: acide 6-(C-β-D-xylopyranoside)-5-hydroxy-hexanoique ;
- 16.: acide 6-(C-α-D-xylopyranoside)-5-hydroxy-hexanoique ;
- 17.: acide 6-(C-β-D-xylopyranoside)-5-amino-hexanoique ;
- 18.: acide 6-(C-α-D-xylopyranoside)-5-amino-hexanoique ;
- 19.: acide 6-(C-β-D-xylopyranoside)-5-phénylamino-hexanoique ;
- 20.: acide 6-(C-α-D-xylopyranoside)-5-phénylamino-hexanoique ;
- 21.: 1-(C-β-D-xylopyranoside)-hexane-2,6-diol ;
- 22.: 1-(C-α-D-xylopyranoside)-hexane-2,6-diol ;
- 23.: acide 5-(C-β-D-xylopyranoside)-4-céto-pentanoique ;
- 24.: acide 5-(C-α-D-xylopyranoside)-4-céto-pentanoique ;
- 25.: acide 5-(C-β-D-xylopyranoside)-4-hydroxy-pentanoique ;
- 26.: acide 5-(C-α-D-xylopyranoside)-4-hydroxy-pentanoique ;
- 27.: acide 5-(C-β-D-xylopyranoside)-4-amino-pentanoique ;
- 28.: acide 5-(C-α-D-xylopyranoside)-4-amino-pentanoique ;
- 29.: acide 5-(C-β-D-xylopyranoside)-4-phénylamino-pentanoique ;
- 30.: acide 5-(C-α-D-xylopyranoside)-4-phénylamino-pentanoique ;
- 31.: 1-(C-β-D-xylopyranoside)-pentane-2,5-diol ;
- 32.: 1-(C-α-D-xylopyranoside)-pentane-2,5-diol ;
- 33.: 1-(C-β-D-fucopyranoside)-propane-2-one ;
- 34.: 1-(C-α-D-fucopyranoside)-propane-2-one ;
- 35.: 1-(C-β-L-fucopyranoside)-propane-2-one ;
- 36.: 1-(C-α-L-fucopyranoside)-propane-2-one ;
- 37.: 1-(C-β-D-fucopyranoside)-2-hydroxy-propane ;
- 38.: 1-(C-α-D-fucopyranoside)-2-hydroxy-propane ;
- 39.: 1-(C-β-L-fucopyranoside)-2-hydroxy-propane ;
- 40.: 1-(C-α-L-fucopyranoside)-2-hydroxy-propane ;
- 41.: 1-(C-β-D-fucopyranoside)-2-amino-propane ;
- 42.: 1-(C-α-D-fucopyranoside)-2-amino-propane ;
- 43.: 1-(C-β-L-fucopyranoside)-2-amino-propane ;
- 44.: 1-(C-α-L-fucopyranoside)-2-amino-propane ;
- 45.: 1-(C-β-D-fucopyranoside)-2-phénylamino-propane ;
- 46.: 1-(C-α-D-fucopyranoside)-2-phénylamino-propane ;
- 47.: 1-(C-β-L-fucopyranoside)-2-phénylamino-propane;
- 48.: 1-(C-α-L-fucopyranoside)-2-phénylamino-propane ;
- 49.: ester éthylique de l'acide 3-méthyl-4-(C-β-D-fucopyranoside)-butyrique ;
- 50.: ester éthylique de l'acide 3-méthyl-4-(C-α-D-fucopyranoside)-butyrique ;
- 51.: ester éthylique de l'acide 3-méthyl-4-(C-β-L-fucopyranoside)-butyrique ;
- 52.: ester éthylique de l'acide 3-méthyl-4-(C-α-L-fucopyranoside)-butyrique ;
- 53.: acide 6-(C-β-D-fucopyranoside)-5-céto-hexanoique ;
- 54.: acide 6-(C-α-D-fucopyranoside)-5-céto-hexanoique ;
- 55.: acide 6-(C-β-L-fucopyranoside)-5-céto-hexanoique ;
- 56.: acide 6-(C-α-L-fucopyranoside)-5-céto-hexanoique ;
- 57.: acide 6-(C-β-D-fucopyranoside)-5-hydroxy-hexanoique ;
- 58.: acide 6-(C-α-D-fucopyranoside)-5-hydroxy-hexanoique ;
- 59.: acide 6-(C-β-L-fucopyranoside)-5-hydroxy-hexanoique ;
- 60.: acide 6-(C-α-L-fucopyranoside)-5-hydroxy-hexanoique ;
- 61.: acide 6-(C-β-D-fncopyranoside)-5-amino-hexanoique ;
- 62.: acide 6-(C-α-D-fucopyranoside)-5-amino-hexanoique ;
- 63.: acide 6-(C-β-L-fucopyranoside)-5-amino-hexanoique ;
- 64.: acide 6-(C-α-L-fucopyranoside)-5-amino-hexanoique ;
- 65.: 1-(C-β-D-fucopyranoside)-hexane-2,6-diol ;
- 66.: 1-(C-α-D-fucopyranoside)-hexane-2,6-diol ;
- 67.: 1-(C-β-L-fucopyranoside)-hexane-2,6-diol ;
- 68.: 1-(C-α-L-fucopyranoside)-hexane-2,6-diol ;
- 69.: acide 5-(C-β-D-fucopyranoside)-4-céto-pentanoique ;
- 70.: acide 5-(C-α-D-fucopyranoside)-4-céto-pentanoique ;
- 71.: acide 5-(C-β-L-fucopyranoside)-4-céto-pentanoique ;
- 72.: acide 5-(C-α-L-fucopyranoside)-4-céto-pentanoique ;
- 73.: acide 5-(C-β-D-fucopyranoside)-4-hydroxy-pentanoique ;
- 74.: acide 5-(C-α-D-fucopyranoside)-4-hydroxy-pentanoique ;
- 75.: acide 5-(C-β-L-fucopyranoside)-4-hydroxy-pentanoique ;
- 76.: acide 5-(C-α-L-fucopyranoside)-4-hydroxy-pentanoique ;
- 77.: acide 5-(C-β-D-fucopyranoside)-4-amino-pentanoique ;
- 78.: acide 5-(C-α-D-fucopyranoside)-4-amino-pentanoique
- 79.: acide 5-(C-β-L-fucopyranoside)-4-amino-pentanoique ;
- 80.: acide 5-(C-α-L-fucopyranoside)-4-amino-pentanoique ;
- 81.: 1-(C-β-D-fucopyranoside)-pentane-2,5-diol ;
- 82.: 1-(C-α-D-fucopyranoside)-pentane-2,5-diol ;
- 83.: 1-(C-β-L-fucopyranoside)-pentane-2,5-diol,
- 84.: 1-(C-α-L-fucopyranoside)-pentane-2,5-diol ;
- 85.: 1-(C-β-D-glucopyranosyl)-2-hydroxy-propane ;
- 86.: 1-(C-α-D-glucopyranosyl)-2-hydroxy-propane ;
- 87.: 1-(C-β-D-glucopyranosyl)-2-amino-propane ;
- 88.: 1-(C-α-D-glucopyranosyl)-2-amino-propane ;
- 89.: 1-(C-β-D-glucopyranosyl)-2-phénylamino-propane ;
- 90.: 1-(C-α-D-glucopyranosyl)-2-phénylamino-propane ;
- 91.: ester éthylique de l'acide 3-méthyl-4-(C-β-D-glucopyranosyl)-butyrique ;
- 92.: ester éthylique de l'acide 3-méthyl-4-(C-α-D-glucopyranosyl)-butyrique ;
- 93.: acide 6-(C-β-D-glucopyranosyl)-5-céto-hexanoique ;
- 94.: acide 6-(C-α-D-glucopyranosyl)-5-céto-hexanoique ;
- 95.: acide 6-(C-β-D-glucopyranosyl)-5-hydroxy-hexanoique ;
- 96.: acide 6-(C-α-D-glucopyranosyl)-5-hydroxy-hexanoique ;
- 97.: acide 6-(C-β-D-glucopyranosyl)-5-amino-hexanoique ;
- 98.: acide 6-(C-α-D-glucopyranosyl)-5-amino-hexanoique ;
- 99.: acide 6-(C-β-D-glucopyranosyl)-5-phénylamino-hexanoique ;
- 100.: acide 6-(C-α-D-glucopyranosyl)-5-phénylamino-hexanoique ;
- 101.: 1-(C-β-D-glucopyranosyl)-hexane-2,6-diol ;
- 102.: 1-(C-α-D-glucopyranosyl)-hexane-2,6-diol ;
- 103.: acide 6-(C-β-D-glucopyranosyl)-5-céto-pentanoique ;
- 104.: acide 6-(C-α-D-glucopyranosyl)-5-céto-pentanoique ;
- 105.: acide 6-(C-β-D-glucopyranosyl)-5-hydroxy-pentanoique ;
- 106.: acide 6-(C-α-D-glucopyranosyl)-5-hydroxy-pentanoique ;
- 107.: acide 6-(C-β-D-glucopyranosyl)-5-amino-pentanoique ;
- 108.: acide 6-(C-α-D-glucopyranosyl)-5-hydroxy-pentanoique ;
- 109.: acide 6-(C-β-D-glucopyranosyl)-5-phénylamino-pentanoique ;
- 110.: acide 6-(C-α-D-glucopyranosyl)-5-phénylamino-pentanoique ;
- 111.: 1-(C-β-D-glucopyranosyl)-pentane-2,5-diol ;
- 112.: 1-(C-α-D-glucopyranosyl)-pentane-2,5-diol ;
- 113.: 1-(C-β-D-galactopyranosyl)-2-hydroxy-propane ;
- 114.: 1-(C-α-D-galactopyranosyl)-2-hydroxy-propane ;
- 115.: 1-(C-β-D-galactopyranosyl)-2-amino-propane ;
- 116.: 1-(C-α-D-galactopyranosyl)-2-amino-propane ;
- 117.: 1-(C-β-D-galactopyranosyl)-2-phénylamino-propane ;
- 118.: 1-(C-α-D-galactopyranosyl)-2-phénylamino-propane ;
- 119.: ester éthylique de l'acide 3-méthyl-4-(β-D-galactopyranosyl)-butyrique ;
- 120.: ester éthylique de l'acide 3-méthyl-4-(α-D-galactopyranosyl)-butyrique ;
- 121.: acide 6-(C-β-D-galactopyranosyl)-5-céto-hexanoique ;
- 122.: acide 6-(C-α-D-galactopyranosyl)-5-céto-hexanoique ;
- 123.: acide 6-(C-β-D-galactopyranosyl)-5-hydroxy-hexanoique ;
- 124.: acide 6-(C-α-D-galactopyranosyl)-5-hydroxy-hexanoique ;
- 125.: acide 6-(C-β-D-galactopyranosyl)-5-amino-hexanoique ;
- 126.: acide 6-(C-α-D-galactopyranosyl)-5-amino-hexanoique ;
- 127.: acide 6-(C-β-D-galactopyranosyl)-5-phénylamino-hexanoique ;
- 128.: acide 6-(C-α-D-galactopyranosyl)-5-phénylamino-hexanoique ;
- 129.: 1-(C-β-D-galactopyranosyl)-hexane-2,6-diol ;
- 130.: 1-(C-α-D-galactopyranosyl)-hexane-2,6-diol ;
- 131.: acide 6-(C-β-D-galactopyranosyl)-5-céto-pentanoique ;
- 132.: acide 6-(C-α-D-galactopyranosyl)-5-céto-pentanoique ;
- 133.: acide 6-(C-β-D-galactopyranosyl)-5-hydroxy-pentanoique ;
- 134.: acide 6-(C-α-D-galactopyranosyl)-5-hydroxy-pentanoique ;
- 135.: acide 6-(C-β-D-galactopyranosyl)-5-amino-pentanoique ;
- 136.: acide 6-(C-α-D-galactopyranosyl)-5-amino-pentanoique ;
- 137.: acide 6-(C-β-D-galactopyranosyl)-5-phénylamino-pentanoique ;
- 138.: acide 6-(C-α-D-galactopyranosyl)-5-phénylamino-pentanoique ;
- 139.: 1-(C-β-D-galactopyranosyl)-pentane-2,6-diol ;
- 140.: 1-(C-α-D-galactopyranosyl)-pentane-2,6-diol ;
- 141.: 1-(C-β-D-fucofuranosyl)-propane-2-one ;
- 142.: 1-(C-α-D-fucofuranosyl)-propane-2-one ;
- 143.: 1-(C-β-L-fucofuranosyl)-propane-2-one ;
- 144.: 1-(C-α-L-fucofuranosyl)-propane-2-one ;
- 145.: 3'-(acétamido-C-β-D-glucopyranosyl)-propane-2'-one ;
- 146.: 3'-(acétamido-C-α-D-glucopyranosyl)-propane-2'-one ;
- 147.: 1-(acétamido-C-β-D-glucopyranosyl)-2-hydroxyl-propane ;
- 148.: 1-(acétamido-C-β-D-glucopyranosyl)-2-amino-propane ;
- 149.: 1-(acétamido-C-β-D-glucopyranosyl)-2-phénylamino-propane ;
- 150.: 1-(acétamido-C-α-D-glucopyranosyl)-2-phénylamino-propane ;
- 151.: ester éthylique de l'acide 3-méthyl-4-(acétamido-C-β-D-glucopyranosyl)-butyrique ;
- 152.: ester éthylique de l'acide 3-méthyl-4-(acétamido-C-α-D-glucopyranosyl)-butyrique ;
- 153.: acide 6-(acétamido-C-β-D-glucopyranosyl)-5-céto-hexanoique ;
- 154.: acide 6-(acétamido-C-α-D-glucopyranosyl)-5-céto-hexanoique ;
- 155.: acide 6-(acétamido-C-β-D-glucopyranosyl)-5-hydroxy-hexanoique ;
- 156.: acide 6-(acétamido-C-α-D-glucopyranosyl)-5-hydroxy-hexanoique ;
- 157.: acide 6-(acétamido-C-β-D-glucopyranosyl)-5-amino-hexanoique ;
- 158.: acide 6-(acétamido-C-α-D-glucopyranosyl)-5-amino-hexanoique ;
- 159.: acide 6-(acétamido-C-β-D-glucopyranosyl)-5-phénylamino-hexanoique ;
- 160.: acide 6-(acétamido-C-α-D-glucopyranosyl)-5-phénylamino-hexanoique ;
- 161.: 1-(acétamido-C-β-D-glucopyranosyl)-hexane-2,6-diol ;
- 162.: 1-(acétamido-C-α-D-glucopyranosyl)-hexane-2,6-diol ;
- 163.: acide 6-(acétamido-C-β-D-glucopyranosyl)-5-céto-pentanoique ;
- 164.: acide 6-(acétamido-C-α-D-glucopyranosyl)-5-céto-pentanoique ;
- 165.: acide 6-(acétamido-C-β-D-glucopyranosyl)-5-hydroxy- pentanoique ;
- 166.: acide 6-(acétamido-C-α-D-glucopyranosyl)-5-hydroxy- pentanoique ;
- 167.: acide 6-(acétamido-C-β-D-glucopyranosyl)-5-amino- pentanoique ;
- 168.: acide 6-(acétamido-C-α-D-glucopyranosyl)-5-amino- pentanoique ;
- 169.: acide 6-(acétamido-C-β-D-glucopyranosyl)-5-phénylamino- pentanoique ;
- 170.: acide 6-(acétamido-C-α-D-glucopyranosyl)-5-phénylamino- pentanoique ;
- 171.: 1-(acétamido-C-β-D-glucopyranosyl)-pentane-2,5-diol ;
- 172.: 1-(acétamido-C-α-D-glucopyranosyl)-pentane-2,5-diol.

A titre illustratif et non limitatif des dérivés C-glycoside convenant plus particulièrement à l'invention, on peut notamment citer les dérivés suivants :
- le C-β-D-xylopyranoside-n-propane-2-one,
- le C-α- D-xylopyranoside-n-propane-2-one,
- le C-β-D-xylopyranoside-2-hydroxy-propane,
- le C-α- D-xylopyranoside-2-hydroxy-propane,
- la 1-(C-β-D-fucopyranoside)-propane-2-one,
- la 1-(C-α-D-fucopyranoside)-propane-2-one,
- la 1-(C-β-L-fucopyranoside)-propane-2-one,
- la 1-(C-α-L-fucopyranoside)-propane-2-one,
- le 1-(C-β-D-fucopyranoside)-2-hydroxy-propane,
- le 1-(C-α-D-fucopyranoside)-2-hydroxy-propane,
- le 1-(C-β-L-fucopyranoside)-2-hydroxy-propane,
- le 1-(C-α-L-fucopyranoside)-2-hydroxy-propane,
- le 1-(C-β-D-glucopyranosyl)-2-hydroxyl-propane,
- le 1-(C-α-D-glucopyranosyl)-2-hydroxyl-propane,
- le 1-(C-β-D-galactopyranosyl)-2-hydroxyl-propane,
- le 1-(C-α-D-galactopyranosyl)-2-hydroxyl-propane
- la 1-(C-β-D-fucofuranosyl)-propane-2-one,
- la 1-(C-α-D-fucofuranosyl)-propane-2-one
- la 1-(C-β-L-fucofuranosyl)-propane-2-one,
- la 1-(C-α-L-fucofuranosyl)-propane-2-one,
- le C-β-D-maltopyranoside-n-propane-2-one,
- le C-α-D-maltopyranoside-n-propane-2-one
- le C-β-D-maltopyranoside-2-hydroxy-propane,
- le C-α-D-maltopyranoside-2-hydroxy-propane, leurs isomères et leurs mélanges.

Selon un mode de réalisation, le C-β-D-xylopyranoside-2-hydroxy-propane ou le C-α-D-xylopyranoside-2-hydroxy-propane, et mieux le C-β-D-xylopyranoside-2-hydroxy-propane, peuvent être avantageusement mis en oeuvre pour la préparation d'une composition selon l'invention.

Selon un mode de réalisation particulier, le dérivé C-glycoside peut être le C-β-D-xylopyranoside-2-hydroxy-propane sous forme d'une solution à 30 % en poids en matière active dans un mélange eau/propylène glycol (60/40 % en poids) tel que le produit fabriqué par CHIMEX sous la dénomination commerciale « MEXORYL SBB^{®} ».

Bien entendu, selon l'invention, un dérivé C-glycoside répondant à la formule (I) peut être utilisé seul ou en mélange avec d'autres dérivés C-glycosides et en toutes proportions.

Un dérivé C-glycoside convenant à l'invention peut notamment être obtenu par la méthode de synthèse décrite dans le document WO 02/051828.

La quantité de dérivé C-glycoside à mettre en oeuvre dans une composition selon l'invention dépend de l'effet cosmétique ou thérapeutique recherché, et peut donc varier dans une large mesure.

L'homme de l'art peut aisément, sur la base de ses connaissances générales, déterminer les quantités appropriées.

Une composition selon l'invention peut comprendre un dérivé C-glycoside à raison d'environ 0,0001 % à environ 25 % en poids de matière active par rapport au poids total de la composition, et en particulier d'environ 0,001 % à environ 10 % en poids de matière active, et plus particulièrement d'environ 0,05 % à environ 5 % en poids de matière active de dérivé C-glycoside par rapport au poids total de la composition.

Le dérivé C-glycoside est utilisé selon l'invention de façon préférée pour la préparation d'une composition formulée pour une administration par voie topique, c'est-à-dire contenant un milieu cosmétiquement ou dermatologiquement acceptable, soit un milieu compatible avec la peau, les ongles, les muqueuses, les tissus, le cuir chevelu et/ou les cheveux.

Il est clair aussi que la quantité efficace d'actif correspond à la quantité nécessaire pour obtenir le résultat désiré, et que la formulation des compositions suivant l'invention dépend de l'usage auquel ces compositions sont destinées.

En particulier, deux grandes catégories de compositions suivant l'invention peuvent être distinguées, en fonction des conditions dans lesquelles elles seront appliquées sur la peau.

La première catégorie correspond aux compositions cosmétiques, c'est-à-dire destinées à être appliquées sur une peau saine afin d'en améliorer l'esthétique et notamment le confort. Une peau saine se définit par l'absence de pathologies telles que des infections, une inflammation, un érythème, ou de blessures telles qu'une brûlure ou une coupure. Cependant, dans cette définition, une peau saine ne signifie pas une peau en parfait état.

En particulier, une peau saine peut présenter des signes de sécheresse qui peuvent être d'origine exogène (la peau devient sèche, par exemple, lors d'une exposition à un air sec et très froid), ou d'origine physiologique endogène (par exemple, au moment de la chute hormonale liée à la ménopause).

Dans un mode de réalisation particulier de l'invention, un dérivé C-glycoside est utilisé pour la préparation d'une composition formulée pour un usage cosmétique, par exemple sur une peau fragile (par exemple une peau de bébé), une peau fragilisée (par exemple une peau d'une personne de grand âge, une peau d'une personne présentant une peau dont la composition du film hydro-lipidique est modifiée), ou une peau sensible. Pour cette utilisation, le dérivé C-glycoside peut être formulé dans une composition telle que décrite précédemment.

La composition cosmétique est ainsi formulée pour être appliquée sur la peau et comprend au moins un dérivé C-glycoside, ladite composition permettant de prévenir, de résorber et/ou de faire disparaître lesdits signes d'inconfort cutané liés à un déficit de la fonction barrière.

Selon l'invention, le dérivé C-glycoside peut aussi être utilisé pour la préparation d'une composition thérapeutique, destinée à améliorer l'état d'une peau lésée, de type peau atopique.

La lésion considérée peut être d'origine quelconque, par exemple infectieuse, allergique, nerveuse ou traumatique. Une composition thérapeutique peut être appliquée directement sur l'endroit lésé ou dans son voisinage, ou encore, dans le cas de pathologies présentant des périodes de crises et des périodes de repos, en un site ayant présenté une lésion, à titre préventif, pour prolonger un état de rémission entre deux crises.

Ainsi, l'invention porte aussi sur l'utilisation d'au moins un dérivé C-glycoside pour la préparation d'une composition formulée pour un usage thérapeutique, par exemple sur une peau lésée de type peau atopique.

La composition sera alors formulée avec un véhicule pharmaceutiquement acceptable.

Selon un mode préféré de réalisation de l'invention, la composition a un pH proche de celui de la peau, compris entre 4 et 7.

Lorsqu'elle est appliquée par voie topique, la composition comprenant au moins un dérivé C-glycoside, peut être appliquée sur le visage, le cou, le cuir chevelu, les muqueuses et les ongles ou tout autre zone cutanée du corps dont les mains et les pieds.

Les compositions selon l'invention sont de préférence formulées sous une forme appropriée pour une administration par voie topique.

Elles comprennent un milieu physiologiquement acceptable.

Par « milieu physiologique acceptable », on entend désigner un milieu compatible avec les matières et/ou les fibres kératiniques d'êtres humains, comme par exemple, de manière non limitative, la peau, les muqueuses, les ongles, le cuir chevelu et/ou les cheveux.

Ce milieu physiologiquement acceptable comprend de l'eau, éventuellement en mélange ou non avec un ou plusieurs solvants organiques tels que des alcools en C₁-C₈, notamment l'éthanol, l'isopropanol, le tert-butanol, le n-butanol, des polyols comme la glycérine, le propylène glycol, le butylène glycol et des éthers de polyol.

Elles se présentent notamment sous forme de solutions aqueuses, hydroalcooliques ou huileuses, de dispersions du type lotion ou sérum, de gels anhydres ou huileux, d'émulsions de consistance liquide ou semi-liquide du type lait, obtenues par dispersion d'une phase grasse dans une phase aqueuse (H/E) ou inversement (E/H), de suspensions ou d'émulsions de consistance molle, semi-solide ou solide du type crème, gel, de micro-émulsions, ou encore de micro-capsules, de micro-particules, ou de dispersions vésiculaires de type ionique et/ou non ionique. Ces compositions sont préparées selon les méthodes usuelles.

Les quantités des différents constituants des compositions utilisées selon l'invention sont celles classiquement utilisées dans les domaines considérés.

Ces compositions constituent notamment des crèmes de protection, de traitement ou de soin pour le visage, pour les mains ou pour le corps, des laits corporels de protection ou de soin, des lotions, gels ou mousses pour le soin de la peau et des muqueuses ou pour le nettoyage de la peau, des masques ou encore des patches.

Les compositions peuvent également consister en des préparations solides constituant des savons ou des pains de nettoyage.

Les compositions utilisées selon l'invention peuvent également contenir un ou plusieurs adjuvants habituels dans les domaines cosmétique et dermatologique, tels que les huiles, les gélifiants hydrophiles ou lipophiles, les actifs hydrophiles ou lipophiles, les agents chélateurs, les conservateurs, les antioxydants, les solvants, les parfums, les charges et les matières colorantes.

Les quantités de ces différents adjuvants sont celles classiquement utilisées dans les domaines considérés, et par exemple de 0,01 % à 20 % du poids total de la composition.

Bien entendu, l'homme de l'art veillera à choisir ce ou ces éventuels additifs et/ou leurs quantités de manière telle que les propriétés avantageuses attachées intrinsèquement à la composition conforme à l'invention, ne soient pas, ou substantiellement pas, altérées par la ou les adjonctions envisagées.

Comme huiles utilisables dans l'invention, on peut citer les huiles minérales (huile de vaseline), les huiles végétales (huile de karité, huile d'amande douce), les huiles animales, les huiles de synthèse, les huiles siliconées (cyclométhicone) et les huiles fluorées (perfluoropolyéthers). On peut aussi utiliser comme matières grasses des alcools gras, des acides gras (acide stéarique), des cires (paraffine, carnauba, cire d'abeilles).

Comme émulsionnants utilisables dans l'invention, on peut citer le Polysorbate 60 et le stéarate de sorbitane vendus respectivement sous les dénominations commerciales Tween 60 et Span 60 par la société ICI. On peut y ajouter des co-émulsionnants tels que le PPG-3 myristyl éther vendu sous la dénomination commerciale Emcol 249-3K par la société WITCO.

Comme solvants utilisables dans l'invention, on peut citer l'eau, les alcools inférieurs, notamment l'éthanol et l'isopropanol, le propylène glycol.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles (xanthane) et les argiles, et, comme gélifiants lipophiles, on peut citer les argiles modifiées comme les bentones, les sols métalliques d'acides gras comme les stéarates d'aluminium, la silice hydrophobe, les polyéthylènes et l'éthylcellulose.

Comme actifs hydrophiles, on peut utiliser les protéines ou les hydrolysats de protéine, les acides aminés, les polyols, l'urée, l'allantoïne, les sucres et les dérivés de sucres, les vitamines hydrosolubles, l'amidon, les extraits bactériens ou végétaux, notamment d'Aloe Vera.

Comme actifs lipophiles, on peut utiliser le tocophérol (vitamine E) et ses dérivés, les acides gras essentiels, les céramides, les huiles essentielles.

Il est en outre possible d'ajouter à la composition selon l'invention un ou plusieurs filtres solaires complémentaires, actifs dans l'UV-A et/ou l'UV-B, hydrophiles ou lipophiles, comportant éventuellement une fonction sulfonique. Le filtre solaire est de préférence choisi parmi les filtres organiques et/ou les filtres minéraux.

Comme filtres organiques, on peut notamment citer les dérivés cinnamiques, les dérivés salicyliques, les dérivés du camphre, les dérivés de triazine, les dérivés de la benzophénone, les dérivés du dibenzoylméthane, les dérivés de β- β-diphénylacrylate, les dérivés de l'acide p-aminobenzoïque, les polymères filtres et silicones filtres décrits dans la demande WO 93/04665 ou encore les filtres organiques décrits dans la demande de brevet EP-A 0 487 404.

Comme filtres minéraux, on peut notamment citer des pigments ou bien encore des pigments présentant une taille moyenne des particules primaires généralement comprise entre 5 nm et 100 nm, de préférence entre 10 et 50 nm d'oxydes métalliques enrobés ou non, comme par exemple des pigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflection et/ou diffusion) du rayonnement UV. Des agents d'enrobage classiques sont par ailleurs l'alumine et/ou le stéarate d'aluminium. De tels pigments d'oxydes métalliques, enrobés ou non enrobés, sont en particulier décrits dans les demandes de brevets EP-A-0 518 772 et EP-A-0 518 773.

Comme exemples de filtres solaires complémentaires actifs dans l'UV-A et/ou l'UV-B, on peut citer :
- l'acide p-aminobenzoïque,
- le p-aminobenzoate oxyéthyléné (25 mol),
- le p-diméthylaminobenzoate de 2-éthylhexyle,
- le p-aminobenzoate d'éthyle N-oxypropyléné,
- le p-aminobenzoate de glycérol,
- le salicylate d'homomenthyle,
- le salicylate de 2-éthylhexyle,
- le salicylate de triéthanolamine,
- le salicylate de 4-isopropylbenzyle,
- le 4-ter-butyl-4'-méthoxy-dibenzoylméthane (PARSOL 1789 de GIVAUDAN ROURE)
- le p-méthoxycinnamate de 2-éthylhexyle (PARSOL MCX de GIVAUDAN ROURE)
- le 4-isopropyl-dibenzoylméthane (EUSOLEX 8020 de MERCK),
- l'anthranilate de menthyle,
- le 2-éthylhexyl-2-cyano-3,3'-diphénylacrylate, (UVINUL N539 de BASF),
- l'éthyl-2-cyano-3,3'-diphénylacrylate,
- l'acide 2-phényl benzimidazole 5-sulfonique et ses sels,
- le 3-(4'-triméthylammonium)-benzylidèn-bornan-2-on-méthylsulfate,
- le 2-hydroxy-4-méthoxybenzophénone (UVINUL MS 40 de BASF),
- le 2-hydroxy-4-méthoxybenzophénone-5-sulfonate (UVINUL MS 40 de BASF),
- le 2,4-dihydroxybenzophénone (UVINUL 400 de BASF),
- le 2,2',4,4'-tétrahydroxybenzophénone (UVINUL D 50 de BASF),
- le 2,2'-dihydroxy-4,4'-diméthoxybenzophénone (HELISORB II de NORQUAY),
- le 2-hydroxy-4-n-octoxybenzophénone,
- le 2-hydroxy-4-méthoxy-4'-méthylbenzophénone,
- l'acide α-(2-oxoborn-3-ylidène)-tolyl-4-sulfonique et ses sels,
- le 3-(4'-sulfo)benzylidèn-bornan-2-one et ses sels,
- le 3-(4'-méthylbenzylidène)-d,1-camphre,
- le 3-benzylidène-d,1-camphre,
- l'acide benzène 1,4-di(3-méthylidène-10-camphosulfonique) et ses sels (MEXORYL SX de CHIMEX),
- l'acide urocanique,
- le 2,4-6-tris-[p-(2'-éthylhexyl-1'-oxycarbonyl)anilino]-1,3,5-triazine,
- le 2-[p-(tertiobutylamido)anilino]-4,6-bis [p-(2'-éthylhexyl-1'-oxycarbonyl)anilino]-1,3,5-triazine,
- le 2,4-bis {[4-2-éthyl-hexyloxyl]-2-hydroxyl-phenyl}-6-(4-méthoxy-phenyl)-1,3,5-triazine,
- le polymère de N-(2 et 4)-[2-oxoborn-3-ylidèn)méthyl)benzyl]-acrylamide,
- l'acide 4,4-bis-benzimidazolyl-phénylèn-3,3',5,5'-tétrasulfonique et ses sels,
- le 2,2'-méthylèn-bis-[6-(2H-benzotriazol-2-yl)-4-(1,1,3,3-tétraméthylbutyl) phénol],
- les polyorganosiloxanes à fonction malonate.

L'invention porte également sur un procédé de traitement cosmétique de la peau destiné à améliorer et/ou renforcer la fonction barrière de la peau comprenant au moins une étape consistant à appliquer sur la peau au moins une composition contenant au moins un dérivé C-glycoside, notamment tel que défini précédemment.

Selon un premier mode, le procédé selon l'invention comprend au moins une étape consistant à appliquer sur la peau de personnes présentant une peau fragile ou délicate au moins une composition comprenant au moins un dérivé C-glycoside, notamment tel que défini précédemment.

Selon un autre mode de réalisation, il comprend au moins une étape consistant à appliquer sur la peau de personnes présentant une peau fragilisée, en particulier la peau de personnes d'au moins 60 ans, voire d'au moins 75 ans, au moins une composition comprenant au moins un dérivé C-glycoside, notamment tel que défini précédemment.

Selon un autre mode de réalisation, il comprend au moins une étape consistant à appliquer sur la peau de personnes présentant une peau ou une zone de peau agressée, en particulier la peau rasée du visage ou du corps, au moins une composition comprenant au moins un dérivé C-glycoside, notamment tel que défini précédemment.

Un autre mode consiste encore à appliquer sur la peau de personnes présentant une peau sensible, au moins une composition comprenant au moins un dérivé C-glycoside, notamment tel que défini précédemment.

Les exemples qui figurent ci-après présentent les résultats d'études *in vitro* relatives à l'effet d'un dérivé C-glycoside sur la fonction barrière d'un épiderme humain reconstruit, ainsi que des exemples non limitatifs de compositions selon l'invention utiles pour améliorer et/ou renforcer la fonction barrière.

### EXEMPLES

Le dérivé C-glycoside utilisé est le C-β-D-xylopyranoside-2-hydroxy-propane commercialisé sous la dénomination MEROXYL^{®} de CHIMEX. Il se présente sous la forme d'une solution à 30 % en poids en matière active dans un mélange eau/1,2propanediol 60/40.

### Exemple 1

### Etude de la pénétration de la caféine sur un épiderme humain reconstruit

L'efficacité de la fonction barrière d'une peau peut être évaluée en mesurant le passage à travers celle-ci d'une molécule marquée, comme par exemple de la caféine radiomarquée.

Une peau présentant une fonction barrière améliorée est ainsi caractérisée en ce qu'elle ralentit, voire diminue, le passage de ladite molécule à travers son épiderme.

Deux lots d'épidermes reconstruits (Episkin J13) (lots 1 et 2) sont traités pendant 5 jours avec le produit C-β-D-xylopyranoside-n-propan-2-one qui est appliqué dans le milieu de culture à la concentration finale de 0,2 % et 0,04 % en poids/volume.

La vitamine C est utilisée comme molécule de référence à la concentration de 200 µg/ml, en traitement systémique.

Après 5 jours de culture, 100 µl à 2 UCi/ml soit 0,04 mM de [¹⁴C]-caféine (caféine radiomarquée) et 0,35 mM de caféine froide (caféine non-radiomarquée) sont déposés en surface et des cinétiques de prélèvement dans le milieu sont réalisées à 8 temps différents.

Les résultats sont exprimés en % de passage par rapport à un témoin non traité.

| | | **lot 1 d'épiderme reconstruit** | | | **lot 2 d'épiderme reconstruit** | | |
|---|---|---|---|---|---|---|---|
| Prélèvement au temps T= | Témoin | Vitamine C 200 µg/ml | C-β-D-xylopyranoside-n-propan-2-one | | Vitamine C 200 µg/ml | C-β-D-xylopyranoside-n-propan-2-one | |
| | | | 0,2 % MA | 0,04 % MA | | 0,2 % MA | 0,04 % MA |
| **1 h** | 100 | - | | | 117 | 162 | 143 |
| **1 h 30** | 100 | 81 | 66 | 52 | - | - | - |
| **2 h** | 100 | - | - | - | 77 * | 61 * | 65 * |
| **3 h** | 100 | 95 | 94 | 66 | 84 | 82 | 92 |
| **4 h** | 100 | 81 | 59* | 64* | 86* | 67* | 78* |
| **5 h** | 100 | 80* | 63* | 73* | 71* | 75* | 84 |
| **6 h** | 100 | 86 | 63* | 70* | 94 | 75* | 80* |
| **7 h** | 100 | 75* | 66* | 69* | 95 | 84* | 91 |
| **8 h** | 100 | - | - | - | 96 | 80* | 88* |
| **9 h** | 100 | 85 | 79* | 77* | 85 | 88 | 93 |
| **10h** | 100 | - | - | - | 95 | 80* | 85* |
| **24 h** | 100 | 85 | 70* | 75* | 99 | 95 | 99 |

| | | | | | | | |
|---|---|---|---|---|---|---|---|
| * *: valeurs statistiquement significatives par rapport au témoin* MA : matière active | | | | | | | |

Dans ces conditions, le produit C-β-D-xylopyranoside-n-propan-2-one ralentit le passage de la caféine radiomarquée à travers des épidermes reconstruits, traduisant ainsi une amélioration de la fonction barrière des épidermes reconstruits.

Ce ralentissement est plus important avec le produit C-β-D-xylopyranoside-n-propan-2-one qu'avec de la vitamine C, utilisée comme molécule de référence dans ce test.

Les résultats ont été validés sur 2 lots d'épiderme reconstruit différents.

### Exemple 2 : Crème de nuit

| **Noms** | **% poids** |
|---|---|
| Stéarate de glycéryle SE | 3,00 |
| Alcool myristique | 0,10 |
| Alcool stéarylique | 0,10 |
| Hydroxyde de sodium | 0,05 |
| Myristate de myristyle | 2,00 |
| Alcool cétylique | 3,80 |
| Cyclopentasiloxane | 5,00 |
| Acide myristique | 0,036 |
| Acide palmitique | 0,528 |
| Acide stéarique | 0,636 |
| Polyisobutène hydrogéné | 8,50 |
| Cire de Candelilla | 0,90 |
| Biotine | 0,04 |
| Vaseline | 4,00 |
| Stéarate de PEG-40 (Myrj 52 P) | 2,00 |
| Hydratants | Qs |
| C-β-D-xylopyranoside-2-hydroxy-propane à 30 % en poids en matière active dans un mélange eau/1,2propanediol 60/40 | 3 % (MA) |
| Conservateurs | Qs |
| Eau | Qsp 100 |

| | |
|---|---|
| MA : Matière active | |

### Exemple 4 : Sérum

| **Noms** | **% poids** |
|---|---|
| Butylène glycol | 5,00 |
| Gomme de xanthane | 0,25 |
| Alcool stéarylique | 1,30 |
| Laureth-7 | 0,07 |
| C-β-D-xylopyranoside-2-hydroxy-propane à 30 % en poids en matière active dans un mélange eau/1,2propanediol 60/40 | 1,80 % (MA) |
| Cyclohexasiloxane | 10,00 |
| Glycérine | 5,00 |
| PEG-20 methyl glucose sesquistearate | 2,00 |
| Ceteareth-20 | 0,70 |
| Isoparaffine C13-14 | 0,21 |
| Huile de noyau de prunus armeniaca (abricot) | 6,00 |
| Méthyl glucose sesquistearate | 2,00 |
| Polyacrylamide | 0,40 |
| Beurre de graine de Shorea robusta | 2,00 |
| Venuceane de Sederma (Thermus thermophillus ferment) | 2,00 |
| Conservateurs | Qs |
| Eau | Qsp 100 |

| | |
|---|---|
| MA : Matière active | |

### Exemple 5 : Crème de jour

| **Ingredients** | **% poids** |
|---|---|
| Acide stéarique | 3,0 |
| Mélange de mono-stéarate de glycéryle et stéarate de polyéthylène glycol (100 OE) | 2,5 |
| Stéarate de polyéthylène glycol (20 OE) | 1,0 |
| Cyclohexadiméthylsiloxane | 10 |
| Huiles végétales | 7 |
| Huiles synthétiques | 6 |
| Charges | 3 |
| Conservateurs | Qs |
| Diméthylsiloxane oxyéthyléné (16 OE) à extrémités méthoxy | 1,0 |
| Gomme de silicone | 0,2 |
| Copolymère acrylique en émulsion inverse Simulgel 600 de SEPPIC) | 1,7 |
| Alcool stéarylique | 1,0 |
| C-β-D-xylopyranoside-2-hydroxy-propane à 30 % en poids en matière active dans un mélange eau/1,2propanediol 60/40 | 10** |
| Eau | Qsp 100 |

| | |
|---|---|
| ** exprimé en % en poids de solution commerciale | |

Ces compositions sont préparées de manière classique pour l'homme du métier.

## Revendications

1. Utilisation cosmétique d'au moins un dérivé C-glycoside dans une composition, pour améliorer et/ou renforcer la fonction barrière de la peau.

2. Utilisation cosmétique d'au moins un dérivé C-glycoside dans une composition, pour améliorer et/ou renforcer la protection de la peau vis-à-vis d'agressions extérieures.

3. Utilisation selon l'une des revendications 1 ou 2, **caractérisée en ce que** ledit dérivé C-glycoside et/ou ladite composition est destiné à prévenir et/ou diminuer l'inconfort cutané d'une peau, en particulier induit par un stress exogène d'origine chimique, environnementale, mécanique et/ou un stress endogène.

4. Utilisation selon la revendication 3, **caractérisée en ce que** l'inconfort cutané est **caractérisé par** des tiraillements, des picotements, des échauffements et/ou des démangeaisons.

5. Utilisation selon l'une quelconque des revendications précédentes, **caractérisée en ce que** ledit dérivé C-glycoside et/ou ladite composition est destiné à améliorer et/ou renforcer la fonction barrière d'une peau choisie parmi une peau fragile, une peau fragilisée, une peau agressée et/ou une peau sensible.

6. Utilisation d'au moins un dérivé C-glycoside pour la préparation d'une composition destinée à améliorer et/ou renforcer la fonction barrière d'une peau lésée, en particulier d'une peau atopique.

7. Utilisation selon la revendication 6 pour la préparation d'une composition destinée à prolonger les phases de rémission entre des crises aiguës d'affections dermatologiques.

8. Utilisation selon l'une quelconque des revendications 1 à 7, dans laquelle le dérivé C-glycoside répond à la formule générale (I) suivante : 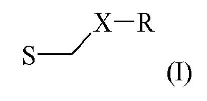 dans laquelle :
- R représente :
- un radical alkyle linéaire, saturé en C₁ à C₂₀, en particulier en C₁ à C₁₀, insaturé en C₂ à C₂₀, en particulier en C₂ à C₁₀, ou un radical alkyle ramifié ou cyclique, saturé ou insaturé, en C₃ à C₂₀, en particulier en C₃ à C₁₀ ;
- un radical hydrofluoro- ou perfluoro-alkyle, linéaire saturé en C₁ à C₂₀, en particulier en C₁ à C₁₀, ou insaturé en C₂ à C₂₀, en particulier en C₂ à C₁₀, ou ramifié ou cyclique, saturé ou insaturé, en C₃ à C₂₀, en particulier en C₃ à C₁₀ ;
la chaîne hydrocarbonée constituant lesdits radicaux pouvant, le cas échéant, être interrompue par 1, 2, 3 ou plus d'hétéroatomes choisis parmi :
- un oxygène,
- un soufre,
- un azote, et
- un silicium,
et pouvant être éventuellement substituée par au moins un radical choisi parmi :
- -OR₄,
- -SR₄,
- -NR₄R₅,
- -COOR₄,
- -CONHR₄,
- -CN,
- un atome d'halogène,
- un radical hydrofluoro- ou perfluoro-alkyle, en C₁ à C₆, et/ou
- un radical cycloalkyle en C₃ à C₈,
avec R₄ et R₅ pouvant représenter, indépendamment l'un de l'autre, un atome d'hydrogène, ou un radical alkyle, perfluoroalkyle ou hydrofluoroalkyle linéaire, saturé en C₁ à C₃₀, notamment en C₁ à C₁₂, ou insaturé en C₂ à C₃₀, notamment en C₂ à C₁₂, ou ramifié ou cyclique, saturé ou insaturé, en C₃ à C₃₀ , notamment en C₃ à C₁₂ ; ou un radical aryle en C₆ à C₁₀,
- X représente un radical choisi parmi les groupements : 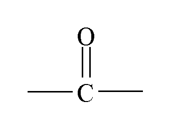 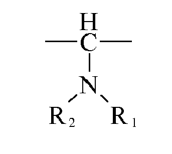 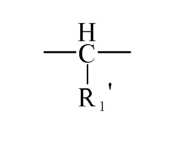 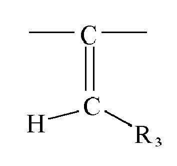 avec R₁, R₂ et R₃ représentant, indépendamment l'un de l'autre, un atome d'hydrogène, ou un radical R, avec R tel que défini précédemment, et R'₁ représentant un atome d'hydrogène, un groupe -OH ou un radical R tel que défini précédemment, R₁ pouvant désigner également un radical aryle en C₆ à C₁₀,
- S représente un monosaccharide ou un polysaccharide comportant jusqu'à 20 unités sucre, en particulier jusqu'à 6 unités sucre, sous forme pyranose et/ou furanose et de série L et/ou D, ledit mono- ou poly-saccharide pouvant être substitué par un groupement hydroxyle obligatoirement libre, et éventuellement une ou plusieurs fonction(s) amine(s) éventuellement protégée(s), et
- la liaison S-CH₂-X représente une liaison de nature C-anomérique, qui peut être α ou β,
ainsi que leurs sels cosmétiquement acceptables, leurs solvates tels que les hydrates et leurs isomères.

9. Utilisation selon la revendication précédente, dans laquelle S représente un monosaccharide choisi parmi le D-glucose, le D-xylose, le L-fucose, le D-galactose, le D-maltose et notamment le D-xylose.

10. Utilisation selon la revendication précédente, dans laquelle X représente un groupement choisi parmi -CO-, -CH(OH)-, -CH(NH₂)-, et préférentiellement un groupement -CH(OH)-.

11. Utilisation selon la revendication précédente, dans laquelle R désigne un radical linéaire en C₁-C₄, notamment C₁-C₃, éventuellement substitué par -OH, -COOH ou -COOR"₂, R"₂ étant un radical alkyle saturé en C₁-C₄, notamment éthyle.

12. Utilisation selon l'une quelconque des revendications précédentes, dans laquelle le dérivé C-glycoside est choisi parmi :
- le C-β-D-xylopyranoside-n-propane-2-one,
- le C-α- D-xylopyranoside-n-propane-2-one,
- le C-β-D-xylopyranoside-2-hydroxy-propane,
- le C-α- D-xylopyranoside-2-hydroxy-propane,
- la 1-(C-β-D-fucopyranoside)-propane-2-one,
- la 1-(C-α-D-fucopyranoside)-propane-2-one,
- la 1-(C-β-L-fucopyranoside)-propane-2-one,
- la 1-(C-α-L-fucopyranoside)-propane-2-one,
- le 1 -(C-β-D-fucopyranoside)-2-hydroxy-propane,
- le 1-(C-α-D-fucopyranoside)-2-hydroxy-propane,
- le 1-(C-β-L-fucopyranoside)-2-hydroxy-propane,
- le 1-(C-α-L-fucopyranoside) -2-hydroxy-propane,
- le 1-(C-β-D-glucopyranosyl)-2-hydroxyl-propane,
- le 1-(C-α-D-glucopyranosyl)-2-hydroxyl-propane,
- le 1-(C-β-D-galactopyranosyl)-2-hydroxyl-propane,
- le 1-(C-α-D-galactopyranosyl)-2-hydroxyl-propane
- la 1-(C-β-D-fucofuranosyl)-propane-2-one,
- la 1-(C-α-D-fucofuranosyl)-propane-2-one
- la 1-(C-β-L-fucofuranosyl)-propane-2-one,
- la 1-(C-α-L-fucofuranosyl)-propane-2-one,
- le C-β-D-maltopyranoside-n-propane-2-one,
- le C-α-D-maltopyranoside-n-propane-2-one
- le C-β-D-maltopyranoside-2-hydroxy-propane,
- le C-α-D-maltopyranoside-2-hydroxy-propane, leurs isomères et leurs mélanges.

13. Utilisation selon l'une quelconque des revendications précédentes dans laquelle le dérivé C-glycoside est choisi parmi le C-β-D-xylopyranoside-2-hydroxy-propane et le C-α-D-xylopyranoside-2-hydroxy-propane, et est plus particulièrement le C-β-D-xylopyranoside-2-hydroxy-propane.

14. Utilisation selon l'une quelconque des revendications précédentes, **caractérisée en ce que** le dérivé C-glycoside est présent à raison d'environ 0,0001 % à environ 25 % en poids, et en particulier d'environ 0,001 % à environ 10 % en poids, et plus particulièrement d'environ 0,05 % à environ 5 % en poids en de matière active de dérivé C-glycoside par rapport au poids total de la composition.

15. Utilisation selon l'une quelconque des revendications précédentes, **caractérisée en ce que** la composition est formulée pour une administration par voie topique.

16. Procédé de traitement cosmétique de la peau destiné à améliorer et/ou renforcer la fonction barrière de la peau, comprenant au moins une étape consistant à appliquer sur la peau de personnes présentant une peau fragile ou délicate au moins une composition comprenant au moins un dérivé C-glycoside.

17. Procédé de traitement cosmétique de la peau destiné à améliorer et/ou renforcer la fonction barrière de la peau, comprenant au moins une étape consistant à appliquer sur la peau de personnes présentant une peau fragilisée, en particulier la peau de personnes d'au moins 60 ans, voire d'au moins 75 ans, au moins une composition comprenant au moins un dérivé C-glycoside.

18. Procédé de traitement cosmétique de la peau destiné à améliorer et/ou renforcer la fonction barrière de la peau, comprenant au moins une étape consistant à appliquer sur la peau de personnes présentant une peau ou une zone de peau agressée, en particulier la peau rasée du visage ou du corps, au moins une composition comprenant au moins un dérivé C-glycoside.

19. Procédé de traitement cosmétique de la peau destiné à améliorer et/ou renforcer la fonction barrière de la peau, comprenant au moins une étape consistant à appliquer sur la peau de personnes présentant une peau sensible, au moins une composition comprenant au moins un dérivé C-glycoside.

20. Procédé de traitement cosmétique selon l'une quelconque des revendications 16 à 19, **caractérisé en ce que** le dérivé C-glycoside est tel que défini selon l'une quelconque des revendications 8 à 13.
